# EUROPEAN PATENT SPECIFICATION

(11) **EP 0 547 558 B1**
(45) Date of publication and mention of the grant of the patent: **19.04.2000**
(21) Application number: 92121318.7
(22) Date of filing: 15.12.1992
(51) Int. Cl.: A61K 31/155

(54) **Use of aminoguanidine for the manufacture of a medicament for inhibiting nitric oxide formation**
Verwendung von Aminoguamidin zur Herstellung eines Arzneimittels zur Unterdrückung der Stickoxidbildung
Utilisation d'aminoguanidine pour la fabrication d'un médicament pour inhiber la formation d'oxyde nitrique

(30) Priority: 16.12.1991 US 807912
(43) Date of publication of application: 23.06.1993
(73) Proprietor: WASHINGTON UNIVERSITY, St. Louis, Missouri 63131 (US)
(72) Inventor: Williamson, Joseph Robert, St. Louis, Missouri 63131 (US); Corbett, John Andrew, St. Louis, Missouri 63139 (US); McDaniel, Michael Lynn, St. Louis, Missouri 63122 (US); Tilton, Ronald Gene, St. Louis, Missouri 63108 (US)
(74) Representative: Goldin, Douglas Michael

(56) References cited:
- EP-A- 0 222 313
- EP-A- 0 339 496
- US-A- 3 541 218
- PHARM. ZTG. vol. 135, no. 26, 28 June 1990, pages 9 - 22; HELMUT R. HENRICHS: 'Diabetes mellitus - ein Krankheitssyndrom und seine Behandlung'
- SCIENCE vol. 232, no. 4758, June 1986, pages 1629 - 1632; M.BROWNLEE ET AL.: 'Aminoguanidine prevents diabetes-induced arterial wall protein cross-linking'
- DIABETES CARE vol. 13, no. 11, November 1990, pages 1180 - 1185; HELEN VLASSARA: 'Chronic diabetic complications and tissue glycosylation'
- J.CLIN.INVEST. vol. 87, no. 2, 1991, pages 432 - 438; RICHARD BUCALA ET AL.: 'Advanced glycosylation products quench nitric oxide and mediate defective endothelium-dependent vasodilatation in experimental diabetes'
- DIABETES vol. 41, no. 8, 1992, pages 897 - 903; JOHN A. CORBETT ET AL.: 'Does nitric oxide mediate autoimmune destruction of beta-cells?'
- Immunology, Wiley-Liss, New York, 1991, pages 5-7 and 204
- Harrison's Principles of Internal Medicine, McGraw-Hill, New York, 13th edition, pages1630-1687
- Immunology, A Synthesis, E.Golub et al. 1991, p.261-269, 545-574
- Am.J.Kidney Dis., vol. 26, no. 6, 1995, pages 875-888
- Diabetes, vol. 42, suppl. 1, page 106A

## Description

Nitric oxide synthase catalyzes the mixed functional oxidation of L-arginine to L-citrulline and nitric oxide (NO^{·} , 1,2). NO^{·} appears to function as either a signaling or an effector molecule depending on the isoform of the enzyme. The constitutive isoform of nitric oxide synthase produces small amounts of NO^{·} which activate guanylate cyclase resulting in the formation of cGMP which mediates endothelium-dependent relaxation (2) and neural transmission (3). NO^{·} is produced in much larger amounts by the cytokine and endotoxin inducible isoform of nitric oxide synthase, and in macrophages functions as an effector molecule which appears to mediate the cytotoxic actions of macrophages on target cells (4). Since NO^{·} is a potent vasodilator and increases blood flow, and since vasoactive agents (such as histamine and bradykinin), which stimulate NO^{·} production increase both blood flow and vascular permeability, NO^{·} may be a candidate for mediating increases in blood flow and vascular permeability induced by diabetes and elevated glucose (5).

Recently, Interleukin-1 (IL-1) has been shown to induce the expression of the cytokine inducible isoform of nitric oxide synthase in pancreatic islets. The production of NO^{·} has been proposed to be the effector molecule which mediates IL-1's inhibitory affects on islet function (6,7). Generation of an IL-1-induced EPR detectable iron-nitrosyl complex, which is prevented by N^{G}-monomethyl-L-arginine (NMMA), has been used to confirm the formation of nitric oxide by islets (8). Also, the protein synthesis inhibitor, cycloheximide has been shown to block IL-1-induced nitrite formation, cGMP accumulation, and EPR detectable iron-nitrosyl complex formation by islets, thus establishing that IL-1 induces the cytokine inducible isoform of nitric oxide synthase in pancreatic islets (7).

The pathogenesis of diabetic complications has been linked to imbalances in sorbitol, myo-inositol, and 1,2-diacyl-sn-glycerol metabolism, and to non-enzymatic glycation of cellular and extracellular constituents (5). The glycation link is supported by evidence that aminoguanidine, a nucleophilic hydrazine compound, interferes with the formation of these glycation products and also attenuates the development of several diabetes-induced vascular (5,9), neural (10), and collagen changes (11). Bucala et al. (12) recently reported that quenching of NO^{·} in vitro by glycated albumin is attenuated by aminoguanidine (present during exposure of albumin to glycating agents) and suggested that glycation products may impair endothelium-dependent relaxation by attenuating NO^{·} activity.

EP-A-0,222,313 discloses a composition for inhibiting advanced glycosylation which comprises aminoguanidine.

Brown *et al*., Science 1986, vol 232, pp 1629-1632 discloses that aminoguanidine prevents diabetes-induced arterial wall protein cross-linking.

EP-A-0,339,496 discloses aminoguanidine derivatives for use in the prevention or treatment of diabetic complications.

This invention relates to the use of aminoguanidine for the preparation of a medicament for the treatment and/or prevention of disease conditions that are immunologically-mediated and not linked to the complications of diabetes or advanced glycosylation end products.

In particular the invention relates to the use of aminoguanidine wherein the disease conditions are selected from the group consisting of arthritis other than osteoarthritis, neuritis, myocardial infarcts, strokes, collagen diseases other than collagen cross-linking and rejection of transplanted organs.

The invention specifically provides use wherein a medicament suitable for intravenous or subcutaneous administration is prepared.

A remarkable and unexpected feature of aminoguanidine is that while it is equipotent to NMMA (an established inhibitor of both the inducible and constitutive isoforms of nitric oxide synthase) in its ability to inhibit IL-1β induced nitric oxide synthase in cultured Rin m5F cells (a rodent insulinoma cell line), it is only ∼1/40th as potent as NMMA in elevating arterial blood pressure. This can provide a great therapeutic advantage for aminoguanidine since it can enable administration of the drug in doses which inhibit induced nitric oxide synthase in the vasculature and in other tissues affected by disease processes without inhibiting constitutive vascular nitric oxide synthase (which would cause hypertension).

The present discovery of the useful inhibitory activity of aminoguanidine against nitric oxide production suggests further uses of this agent for:

Prevention/treatment of a broad spectrum of acute and chronic diseases which may be linked to production of nitric oxide by activated macrophages and other cells. Examples of such diseases include arthritis (other than osteoarthritis), neuritis, myocardial infarcts, strokes, collagen diseases (other than collagen cross-linking), rejection of transplants organs, and other diseases mediated by immune mechanisms.

### Detailed Description of the Invention

While the specification concludes with claims particularly pointing out and distinctly claiming the subject matter regarded as forming the present invention, it is believed that the invention will be better understood from the following detailed description of preferred embodiments of the invention taken in conjunction with the accompanying drawings in which briefly:
FIG. 1 is a graphical representation which shows in Panel A, the effects of aminoguanidine (AG) on IL-1β-induced nitrite formation; Panel B, cGMP accumulation by Rin m5F cells; and Panel C, IL-1β-induced inhibition of glucose stimulated secretion by islets.
FIG. 2 shows by EPR spectroscopy the effects of aminoguanidine on IL-1-induced iron-nitrosyl complex formation by islets.
FIG. 3 is a graphical representation which shows the effects of aminoguanidine (AG) and N^{G}-monomethyl-L-arginine (NMMA) on mean arterial blood pressure (MAP).
FIG. 4 is a graphical representation which shows the effects of aminoguanidine (AG) and N^{G}-monomethyl-L-arginine (NMMA) on glucose-(Glu) in Panel A, and diabetes-induced increases in vascular albumin permeation in Panel B and blood flow in Panel C.

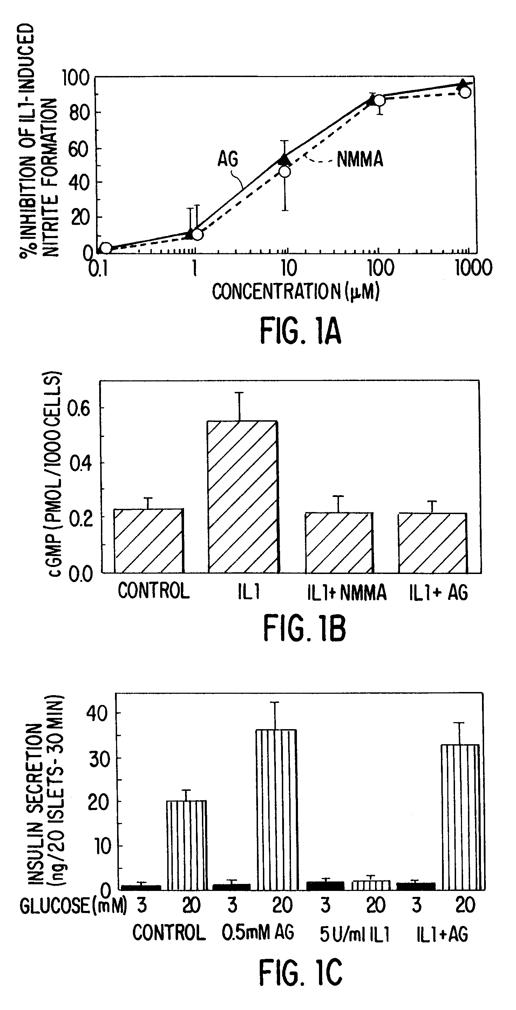

Since aminoguanidine contains strong structural similarities to L-arginine and the competitive inhibitor of nitric oxide synthase, viz. NMMA, in that these compounds contain two chemically equivalent guanidino nitrogen groups, the effects of aminoguanidine on IL-1β-induced formation of nitrite, and cGMP by Rin m5F cells were examined and compared to the effects of NMMA in similar tests. The Rin m5F cell line is an insulinoma cell line of the rodent β-cell that has recently been shown to contain the cytokine-inducible isoform of nitric oxide synthase (13). Figure 1A demonstrates the dose response of aminoguanidine and NMMA on IL-1β-induced formation of nitrite (an oxidation product of nitric oxide) from Rin m5F cells incubated for 18 hrs with 5 units/ml IL-1β ± the indicated concentrations of aminoguanidine or NMMA. Both compounds inhibit IL-1β-induced nitrite production in a dose dependent fashion, in which both compounds inhibit half maximally at a concentration of ∼10 µM.

Next, the effects of aminoguanidine on IL-1β-induced cGMP accumulation, a very sensitive measure of NO^{·} production (7), were examined. Rin m5F cells were treated for 18 hrs with 5 units/ml IL-1β ± 0.5 mM NMMA, or ± 0.5 mM aminoguanidine, followed by a 3 hr incubation with the phosphodiesterase inhibitor isobutyl-methylxanthine (1 mM). Figure 1B demonstrates that IL-1β-induced accumulation of cGMP in Rin m5F cells was completely blocked by aminoguanidine and by NMMA, providing further evidence that aminoguanidine inhibits nitric oxide synthase.

The effect of aminoguanidine on IL-1β-induced inhibition of glucose-stimulated insulin secretion was also examined. Figure 1C demonstrates that glucose-stimulated insulin secretion is significantly inhibited by pretreatment of islets for 18 hrs with IL-1β. Aminoguanidine (0.5 mM) completely prevents this IL-1β-induced inhibition of insulin secretion. Aminoguanidine in the absence of IL-1β slightly potentiates insulin secretion by islets, although this effect is not statistically significant (p>0.075) when compared to insulin secretion by the untreated control islets. NMMA also has been shown to slightly potentiate insulin secretion and completely block IL-1β induced inhibition of insulin secretion (8). The slight stimulation of insulin secretion induced by NMMA and aminoguanidine may be due to the inhibition of both the constitutive and inducible isoforms of nitric oxide synthase (7,8).

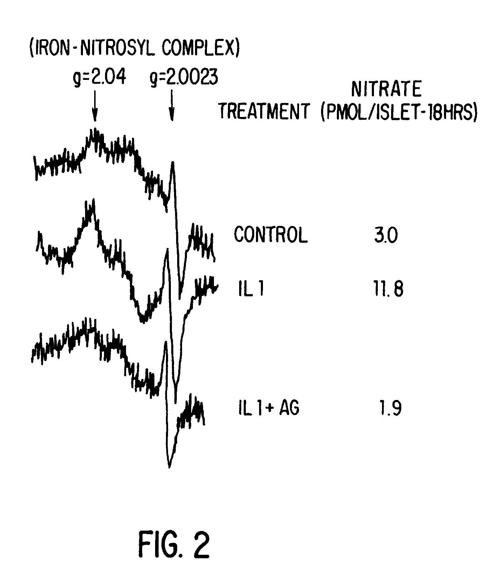

To confirm that aminoguanidine inhibits IL-1β-induced nitric oxide synthase activity, the effects of aminoguanidine on IL-1β-induced formation of a g=2.04 EPR-detectable iron-nitrosyl feature by islets was investigated. Figure 2 demonstrates that aminoguanidine (0.5 mM) completely prevents the formation of this IL-1β induced iron-nitrosyl feature. Also shown in this figure is the concurrent formation of nitrite by the same islets used for EPR spectroscopy. The formation of this g=2.04 iron-nitrosyl EPR-detectable feature has been used previously to confirm the formation of NO^{.} in islets treated with IL-1β (7,8), in activated macrophages (14), and in target cells recognized by activated macrophages (15). The formation of this feature (an iron-dinitrosyl complex) is consistent with the possibility that removal of non-heme iron from iron containing enzymes may be the mechanism by which nitric oxide inhibits insulin secretion (7,14,15). These results confirm that aminoguanidine is a potent inhibitor of the cytokine-inducible isoform of nitric oxide synthase.

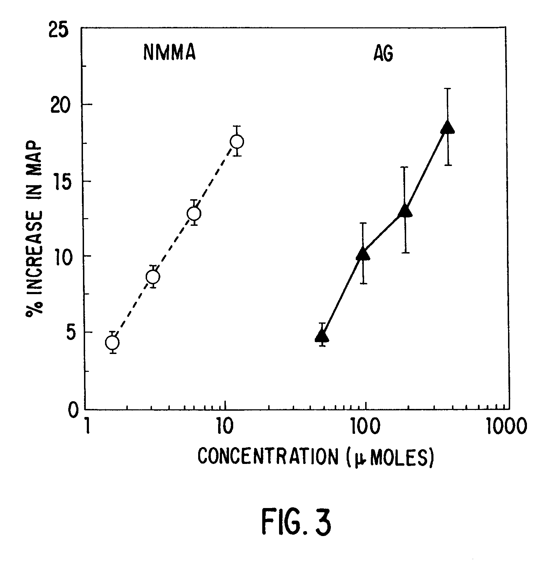

Effects of aminoguanidine on constitutive (vascular) nitric oxide synthase activity were assessed by monitoring mean arterial blood pressure (MAP) changes following intravenous injection of aminoguanidine in anesthetized, normal rats. The dose responses of aminoguanidine and NMMA are shown in Figure 3. Both compounds increase MAP in a dose-dependent fashion, although NMMA is at least 10 fold more potent than aminoguanidine. The difference in sensitivity does not appear to be due to the inhibitory effects of aminoguanidine on diamine oxidase activity (16), since pretreatment with diphenhydramine (an H₁ receptor blocker) has no effect on aminoguanidine-induced increases in MAP (13). These observations suggest that while aminoguanidine and NMMA are equipotent inhibitors of cytokine-induced nitric oxide synthase in vitro, aminoguanidine unexpectedly and advantageously is much less potent than NMMA as an inhibitor of the constitutive isoform of nitric oxide synthase in vivo.

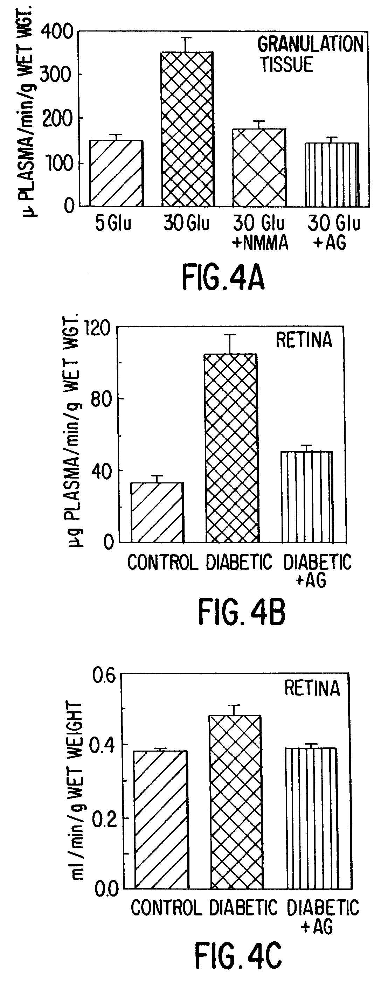

To assess the putative role of NO^{·} in mediating glucose-induced vascular dysfunction, the effects of 1 mM aminoguanidine or 1 mM NMMA on 30 mM glucose-induced increases in vascular clearance of ¹³¹I-albumin were examined in a skin chamber, granulation-tissue model in nondiabetic rats (17). As shown in Figure 4A, exposure of newly forming granulation tissue vessels to 30 mM glucose twice daily for 6-7 days caused a ∼2 fold (P <0.005) increase in ¹³¹I-albumin clearance, which is completely prevented by coadministration of aminoguanidine or NMMA. Similarly, in rats with streptozotocin-induced diabetes of 5 weeks duration, aminoguanidine (50 mg/kg/day injected sc) prevents the 2-4 fold increases in ¹³¹I-albumin clearance in ocular tissues (P <0.001) including the retina (Fig. 4B), sciatic nerve (9), and aorta (13). Likewise, in rats with diabetes of 4 months duration, aminoguanidine prevented blood flow increases (P <0.001) in the retina (Fig. 4C), as well as other ocular tissues and sciatic nerve (13). In these tests aminoguanidine had no effect on blood pressure or on tissue sorbitol and myo-inositol levels (13).

The finding that aminoguanidine and NMMA prevent diabetes- and glucose-induced increases in ¹³¹I-albumin permeation implies a role for NO^{·} in mediating impaired vascular barrier function as well as increased blood flow in diabetes. This interpretation is consistent with evidence that: 1) increased vascular permeability and blood flow induced by vasoactive agents such as histamine and bradykinin in dondiabetic animals are coupled to increased NO^{.} production, and 2) NO^{·} modulates the metabolism and function of the cells in which it is produced (2,4).

The likelihood that normalization of vascular function in these tests reflects inhibition of nitric oxide synthase activity by aminoguanidine rather than prevention of advanced glycation end products is strongly supported by the brief duration (48 hour maximum) of elevated glucose in the granulation tissue model (17), and the prevention by aminoguanidine of 1 mM sorbitol-induced increased vascular albumin permeability in granulation tissue in nondiabetic rats (18). These findings, coupled with evidence that pyruvate and inhibitors of the sorbitol pathway also prevent glucose-induced increased vascular permeability (17,18), suggest that interactions between sorbitol pathway-linked redox imbalances in NAD(P)NAD(P)H⁺ (5), NADPH dependent nitric oxide synthesis, and inhibition of oxidative metabolism by NO^{·} (4) may be important in the pathogenesis of diabetic complications.

In order to further illustrate the invention, the following detailed examples were carried out although it should be understood that the invention is not limited to these specific examples or the details described therein. The results obtained in these examples are shown in the accompanying Figs. 1 to 4.

### Example 1

This example illustrates the effects of aminoguanidine on IL-1β-induced nitrite formation, and cGMP accumulation by Rin m5F cells, and IL-1β-induced inhibition of glucose stimulated insulin secretion by islets. (Fig. 1A). Rin m5F cells, obtained from the Washington University Tissue Culture Support Center, were removed from growth flasks (55-80 million cells/flask) by trypsin/EDTA treatment, and aliquoted into 1 ml Petri dishes (1-2 million Rin m5F cells per condition). Cells were incubated for 18 hrs (under an atmosphere of 95% air and 5% CO₂) in 1 ml of complete CMRL-1066 tissue culture media (CMRL supplemented with 10% heat-inactivated, fetal bovine serum, 2 mM L-glutamine, 50 units/ml penicillin, and 50 µg/ml streptomycin), or complete CMRL-1066 supplemented with aminoguanidine or NMMA. Following incubation, the supernatant was removed and nitrite was determined on 100 µl aliquots by conventional procedures as previously described (8,19). Results are expressed as a mean ± SEM of data from at least 3 independent tests. (Fig. 1B). cGMP accumulation was determined on 8 million Rin m5F cells incubated for 18 hrs in 1 ml of complete CMRL-1066 supplemented with 5 units/ml IL-1β, 0.5 mM aminoguanidine-HCl, 0.5 mM NMMA, or both IL-1β and aminoguanidine-HCl or IL-1β and NNMA as described above. Following the 18 hr treatment the Rin m5F cells were incubated an additional 3 hrs in 1 ml of complete CMRL-1066 containing 1 mM isobutyl-methylxanthine. cGMP formation induced by IL-1β was measured using a conventional cGMP RIA kit commercially available from NEN-DuPont (7). Data is expressed as the mean ± SEM of 3 independent tests. (Fig. 1C). For insulin secretion studies islets were isolated by collagenase digestion from male Sprague-Dawley rats (250-300 g) by conventional procedures as described previously (20). The isolated islets (120/ml) were pretreated in complete CMRL-1066, or complete CMRL-1066 containing 5 units/ml IL-1β, 0.5 mM aminoguanidine-HCl, or IL-1β and aminoguanidine-HCl for 18 hrs at 37°C under an atmosphere of 95% air and 5% CO₂. Following pretreatment the islets were washed 3 times in 1 ml/wash of Krebs-Ringer bicarbonate buffer (KRB: 25 mM Hepes, 115 mM NaCl, 24 mM NaHCO₃, 5 mM KCl, 1 mM MgCl₂, 2.5 mM CaCl₂, pH 7.4) containing 3 mM D-glucose, and 0.5% BSA. Groups of 20 islets were counted into 10 x 75 mm siliconized borosilicate tubes and preincubated for 30 min in 200 µl of the same buffer. The preincubation buffer was removed and glucose-stimulated insulin secretion was initiated by the addition of 200 µl of fresh KRB containing 3 mM D-glucose or 20 mM D-glucose followed by an incubation of 30 min. Both the preincubation and incubation were performed under an atmosphere of 95% air and 5% CO₂ at 37°C. Insulin content of the incubation media was measured by radioimmunoassay. Results are expressed as the mean ± SEM of data from three individual tests. Statistical significance (P <0.05) was determined by the Students t-test.

### Example 2

This example illustrates the effects of aminoguanidine on IL-1-induced iron-nitrosyl complex formation by islets. Isolated islets (2400) were cultured in 3 ml of complete CMRL-1066, or complete CMRL-1066 containing 5 units/ml IL-1β, 0.5 mM aminoguanidine-HCl, or both IL-1β and aminoguanidine-HCL for 18 hrs at 37°C under an atmosphere of 95% air and 5% CO₂. The islets were isolated by centrifugation and frozen at -70°C. EPR spectroscopy was performed at 77 K on the islets using a Varian E-109 spectrometer equipped with a 9.5 GHz microwave bridge. The power was 1 mW, the modulation frequency was 100 kHz, the modulation amplitude was 8 guass, and the microwave frequency was 9.109 GHz. Nitrite was determined as described in Example 1 (Fig. 1A) on 100 µl aliquots of the same islet culture supernatant used for EPR spectroscopy. The data shown in Fig. 2 are representative of 3 individual tests containing at least 2400 islets per condition. The IL-1β-induced iron-nitrosyl g=2.04 complex and the delocalized electron g=2.0023 features (found in all cells) are indicated by arrows.

### Example 3

This example illustrates the effects of aminoguanidine and NMMA on mean arterial blood pressure. Normal male, Sprague-Dawley rats were anesthetized with 100 mg/kg body weight Inactin, the left femoral vein (for tracer injection) and right iliac artery (for monitoring blood pressure) were cannulated with polyethylene tubing filled with heparinized saline, and the trachea was cannulated and connected to a small rodent respirator for continuous ventilatory support. Following stabilization of arterial pressure, increasing amounts of aminoguanidine or NMMA were injected intravenously in separate animals and the peak pressure increase was recorded. Results are shown in Fig. 3 and are expressed as a percent increase in pressure above baseline and represent a mean ± SEM. n = 6 for aminoguanidine and n = 5 for NMMA.

### Example 4

This example illustrates the effects of aminoguanidine and NMMA on glucose- and diabetes-induced increases in vascular albumin permeation and blood flow. (Fig. 4A). Glucose-induced increases in vascular albumin permeation were assessed in a conventional granulation tissue, skin-chamber model (17). Briefly, 2-cm circles of skin were removed from the back on either side of the midline of anesthetized, normal nondiabetic rats. A plastic chamber equipped with stainless steel screwcaps (which can be readily removed to permit addition of pharmacological agents to the new granulation tissue vessels as they form inside the chamber) is sutured to the skin at the margins of the wound. Seven days after inserting the chambers, 1.5 ml of Hepes buffer, pH 7.4 (25 mM Hepes, 137 mM NaCl, 4.2 mM KCl, 3 mM Na₂HPO₄, 0.3 mM L-arginine, 100 µg/ml penicillin G, 10 µg/ml gentamicin) containing 5 mM glucose, or 30 mM glucose, or 30 mM glucose + 1 mM aminoguanidine, or 30 mM glucose + 1 mM NMMA were added to each individual chamber (n = 4 for each test condition). Solutions were prepared fresh daily and were added twice daily to each chamber (9:00 AM and 5:00 PM) for 7 days. Within an hour of the last treatment, albumin clearance was assessed by conventional procedures as previously described (21). (Fig. 4B). Male, Sprague-Dawley rats initially weighing ∼250 g were divided into nondiabetic controls (n = 8), controls treated with 50 mg/kg body weight aminoguanidine (n = 8), untreated diabetics (n = 11), and diabetics treated with the same dose of aminoguanidine (n = 9). Diabetes was induced by i.v. injection of streptozotocin, at which time, rats in groups 2 and 4 were given aminoguanidine subcutaneously once daily. Rats were used for vascular permeability studies after 5 weeks of diabetes. Albumin permeation of retinal vessels was assessed by a conventional quantitative isotope-dilution technique described previously (21) based on the injection of ¹³¹I-BSA (circulation time 10 min) for assessment of vascular albumin permeation and ¹²⁵I-BSA (circulation time 2 min) as a vascular space reference tracer to correct intravascular ¹³¹I-BSA tissue activity. To calculate ¹³¹I-BSA vascular clearance (µg plasma/g tissue wet weight/min), ¹³¹I-BSA activity in the retina was corrected for tracer contained within retinal vessels; the vascular-corrected ¹³¹I-BSA retina activity was divided by a time-averaged ¹³¹I-BSA plasma activity obtained from the plasma samples taken at 1, 5, and 10 min after tracer injection, then by the tracer circulation time (10 min) and normalized per g tissue wet weight. (Fig. 4C). Regional blood flows (n = 7 for each group) were measured after 4 months of diabetes using ⁸⁵Sr-labeled microspheres by conventional techniques as described previously (22). To calculate regional blood flows, the total activity of ⁸⁵Sr in the retina was divided by the total activity of ⁸⁵Sr in the reference blood sample obtained from the withdrawal syringe, then multiplied by the pump withdrawal rate, and expressed as ml/g tissue wet weight per minute.

The aminoguanidine inhibitor of nitric oxide formation described herein can be used for administration to warm blooded mammals by conventional means, preferably in formulations with pharmaceutically acceptable diluents and carriers. The amount of the active inhibitor to be administered must be an effective amount, that is, an amount which is medically beneficial but does not present toxic effects which overweigh the advantages which accompany its use. It would be expected that the adult human daily dosage would normally range upward from about one milligram per kilo of body weight of the drug. A suitable route of administration is orally in the form of capsules, tablets, syrups, elixirs and the like, although parenteral administration also can be used, e.g. intraveneously, intraperitoneally or subcutaneously. Intraveneous administration of the drug in aqueous solution such as physiologic saline is illustrative. Appropriate formulations of the drug in pharmaceutically acceptable diluents and carriers in therapeutic dosage form can be prepared by reference to general texts in the field such as, for example, Remington's Pharmaceutical Sciences, Ed. Arthur Osol. 16th ed., 1980, Mack Publishing Co., Easton, PA.

References cited in parenthesis in the disclosure are appended hereto as follows:
1. D. J. Stuehr, H. J. Cho, N. S. Kwon, M. F. Weise, C. F. Nathan, Proc. Natl. Acad. Sci. USA 88, 7773 (1991).
2. S. Moncada, R. M. J. Palmer, E. A. Higgs, Pharmacol. Reviews 43, 109 (1991).
3. J. Garthwaite, Trends Neurol. Sci. 14:60 (1991).
4. J. B. Hibbs, Jr., et al., in Nitric Oxide from L-Arginine: a Bioregulatory System, S. Moncada and E. Higgs, Eds. Elsevier, New York, (1990) pp 189-223.
5. G. Pugliese, R. G. Tilton, J. R. Williamson, Diabetes/Metabolism Reviews 7, 35 (1991).
6. C. Southern, D. Schulster, I. C. Green, Febs Lett. 276, 42 (1990).
7. J. A. Corbett, J. L. Wang, M. A. Sweetland, J. R. Lancaster, Jr., M. L. McDaniel, Biochemical J. (submitted).
8. J. A. Corbett, J. R. Lancester, Jr., M. A. Sweetland, M. L. McDaniel, J. Biol. Chem. 266, 21351-21354 (1991).
9. J. R. Williamson et al., Diabete & Metab. 16, 3369 (1990). T. Soulis-Liparota, M. Cooper, D. Papazoglou, B. Clarke, G. Jerums, Diabetes 40, 1328 (1991).
10. M. Kihara et al., Proc. Natl. Acad. Sci. USA 88, 6107 (1991).
11. M. Brownlee, A. Cerami, H. Vlassara, N. Engl. J. Med. 318, 1315 (1988). M. Brownlee, H. Vlassara, A. Kooney, P. Ulrich, A. Cerami, Science 232, 1629 (1986).
12. R. Bucala, K. J. Tracey, A. Cerami, J. Clin. Invest. 87, 432 (1991).
13. Unpublished observations.
14. J. R. Lancaster, Jr., J. B. Hibbs, Jr. Proc. Natl. Acad. Sci. USA 87, 1223 (1990).
15. J. C. Drapier, C. Pellat, Y. Henry J. Biol. Chem. 266, 10162 (1991).
16. M. A. Beaven, R. E. Shaff Biochem. Pharmacol. 24, 979 (1975).
17. J. R. Williamson, J. Clin. Invest. 85, 1167 (1990). B. A. Wolf et al., J. Clin. Invest. 87, 31 (1991).
18. K. Chang, W. Allison, J. Harlow, J. R. Williamson, Diabetes 40, 210A (1991).
19. L. C. Green et al., Anal. Biochem. 126, 131 (1982).
20. M. L. McDaniel, J. R. Colca, N. Kotagal, P. E. Lacy, Methods Enzymol. 98, 182 (1983).
21. G. Pugliese et al., Diabetolgia 32, 847 (1990). G. Pugliese et al., Metabol 39, 690 (1990).
22. R. G. Tilton, K. Chang, C. Weigel, C. Kilo, J. R. Williamson, Invest. Ophthalmal. Vis. Sci. 29, 861 (1988).

## Claims

1. Use of aminoguanidine for the preparation of a medicament for the treatment and/or prevention of disease conditions that are immunologically-mediated and not linked to the complications of diabetes or advanced glycosylation end products.

2. Use of aminoguanidine according to claim 1 wherein the disease conditions that are immunologically-mediated are selected from the group consisting of arthritis other than osteoarthritis, neuritis, myocardial infarcts, strokes, collagen diseases other than collagen cross-linking and rejection of transplanted organs.

3. Use of aminoguanidine according to claim 1 or 2, wherein a medicament suitable for intravenous or subcutaneous administration is prepared.

## Patentansprüche

1. Verwendung von Aminoguanidin zur Herstellung eines Arzneimittels zur Behandlung und/oder Vorbeugung von Erkrankungen, die immunologisch begünstigt sind und nicht mit durch Diabetes oder die Endprodukte fortgeschrittener Glykosylierung verursachten Komplikationen zusammenhängen.

2. Verwendung von Aminoguanidin nach Anspruch 1, bei der die immunologisch begünstigten Erkrankungen ausgewählt werden aus der Gruppe, bestehend aus Arthritis, Osteoarthritis ausgenommen, Neuritis, Myocardinfarkten, Schlaganfällen, Kollagenosen, Kollagenfaservernetzung ausgenommen, und Abstoßung transplantierter Organe.

3. Verwendung von Aminoguanidin nach Anspruch 1 oder 2, bei der ein für die intravenöse oder subkutane Verabreichung geeignetes Arzneimittel hergestellt wird.

## Revendications

1. Utilisation d'aminoguanidine pour la préparation d'un médicament destiné au traitement et/ou à la prévention d'états maladifs qui sont à médiation immunologique et non liés aux complications du diabète ou à des produits finaux de la glycosylation avancée.

2. Utilisation d'aminoguanidine selon la revendication 1 dans laquelle les états maladifs qui sont à médiation immunologique sont choisis dans le groupe constitué par les arthrites autres que l'ostéoarthrite, les névrites, les infarctus du myocarde, les attaques, les maladies du collagène autres que la réticulation du collagène et le rejet d'organes transplantés.

3. Utilisation d'aminoguanidine selon la revendication 1 ou 2, dans laquelle un médicament approprié à l'administration intraveineuse ou sous-cutanée est préparé.
